(19) Europäisches Patentamt
European Patent Office
Office européen des brevets

(11) EP 2 925 289 B1

(12) EUROPEAN PATENT SPECIFICATION

(45) Date of publication and mention of the grant of the patent:
**02.05.2018 Bulletin 2018/18**

(21) Application number: **13859379.3**

(22) Date of filing: **20.11.2013**

(51) Int Cl.:
***A61K 8/81*** (2006.01) ***A61K 8/26*** (2006.01)
***A61Q 19/10*** (2006.01)

(86) International application number:
**PCT/CN2013/087522**

(87) International publication number:
**WO 2014/082540 (05.06.2014 Gazette 2014/23)**

# (54) STRUCTURED LIQUIDS CONTAINING REFLECTIVE PARTICLES

STRUKTURIERTE FLÜSSIGKEITEN MIT REFLEKTIERENDEN TEILCHEN

LIQUIDES STRUCTURÉS CONTENANT DES PARTICULES RÉFLÉCHISSANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2012 PCT/CN2012/085554**
**02.04.2013 PCT/CN2013/073673**
**21.05.2013 EP 13168622**

(43) Date of publication of application:
**07.10.2015 Bulletin 2015/41**

(73) Proprietors:
- **Unilever PLC**
**London, Greater London EC4Y 0DY (GB)**
Designated Contracting States:
**CY GB IE MT**
- **UNILEVER N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**

(72) Inventors:
- **HODSON, Emma Louise**
**Bebington**
**Wirral**
**Merseyside CH63 3JW (GB)**
- **JARVIS, Adam Peter**
**Bebington**
**Wirral**
**Merseyside CH63 3JW (GB)**
- **KITA-TOKARCZYK, Katarzyna Elzbieta**
**65812 Bad Soden am Taunus (DE)**
- **LIMER, Adam John**
**Shanghai 200335 (CN)**
- **MERRINGTON, James**
**Bebington**
**Wirral**
**Merseyside CH63 3JW (GB)**

(74) Representative: **Fijnvandraat, Arnoldus et al**
**Unilever Patent Group**
**Colworth House**
**Sharnbrook**
**Bedford MK44 1LQ (GB)**

(56) References cited:
**WO-A1-2006/012465** **WO-A1-2007/090759**
**WO-A1-2011/062805** **WO-A1-2012/054107**
**US-A- 4 529 773** **US-A1- 2008 112 913**
**US-A1- 2008 207 774**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 925 289 B1

## Description

BACKGROUND OF THE INVENTION

Field of the Invention

This invention relates to compositions in the form of structured liquids which comprise light reflective particles and, more particularly, to cleansing compositions such as shampoos, body washes, and other detersive products for personal care applications.

The Related Art

Light reflective particles can be used in structured liquids to provide visual impact to the consumer. Such particles come in a variety of geometries, with common materials being particles in the form of platelets, flakes and powders.

Formulators using such particles are faced with the challenge of keeping the particles suspended and well dispersed in the compositions in which they are employed, as well as avoiding undesirable particle clumping or aggregation during processing. Additionally, formulators need to ensure that the suspensions/dispersions remain stable over time and are not undesirably impacted by changes in temperature such as can occur during product transit and storage.

The structured liquids of interest are frequently in the form of micellar liquids, in particular, liquids that include micellar structures dispersed in an aqueous or other polar phase. In cleansing products for personal care applications, the micellar structures, also known as micelles, are typically aggregates of surfactant molecules. In aqueous or other polar systems, aggregation of surfactant typically results in a structure in which the polar or hydrophilic head of the surfactant molecules forms an outer region that surrounds a core formed from the non-polar or hydrophobic tail region of the surfactant molecules. While often elongated, rod shaped, worm-like, or generally spherical in appearance, micelle geometry is subject to variation and is determined, in part, by choice and level of surfactant.

The structured liquids of interest may also take the form of microemulsions, i.e., optically isotropic and thermodynamically stable dispersions of immiscible liquids stabilized by surfactant, in which the dispersed phase commonly has a droplet size of less than 100 nm.

A significant factor in the appearance of structured liquids containing reflective particles is the manner in which light is reflected back to the observer. Increasing the amount of particles can often give rise to a shinier appearance. Reflective particles tend, however, to be relatively expensive materials. Additionally, increasing the amount of such particles does not ensure that the individual particles will be well dispersed or that the reflective potential of the individual particles will be maximized. That is to say, other than increasing the potential for light reflection, the use of higher levels of reflective particles may not otherwise address the manner in which light is reflected by the individual particles.

Interactions among components of a structured liquid, for example, interactions between structuring materials and micelles, micelles and particles, and particles and structuring materials, can impact the manner in which such particles are dispersed as well as the liquid's appearance and optical properties. Additionally, such interactions can affect the rheological properties of the resulting liquids. Choice and amounts of structuring agent and other components can also impact the optical properties of reflective particles in a structured liquid. Thus, reflective particles that provide a dull appearance in one formulation can give rise to a shinier appearance in another formulation. Achieving desirable optical properties in transparent, semi-transparent or translucent liquids can be especially challenging.

(Meth)acrylic acid polymers are among the structuring materials often used in surfactant-containing cleansing compositions. The chemistry of (meth)acrylic acid polymers is generally compatible with the surfactants typically found in such compositions, i.e., anionic, nonionic, ampthoteric and/or zwitterionic surfactants.

Common structuring materials include polymeric materials known as "carbomers", including, for example the cross-linked polyacrylic acid polymers available from Lubrizol Corporation under the trademark Carbopol®. Another class of (meth)acylic acid polymers are alkali-swellable emulsion (ASE) polymers. ASE polymers include, for example, Aculyn® 38 copolymer from Dow.

Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodyamic polymers commonly give rise to compositions that exhibit shear thinning or non-Newtonian behavior.

Another class of (meth)acrylic acid based rheology modifiers are hydrophobically modified alkali swellable

(HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn® 22 and Aculyn® 28 copolymers from Dow and Aqua SF-1® copolymer from Lubrizol Corporation are among the commonly used HASE materials.

U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1) methacrylic or acrylic acid, (2) methacrylic or acrylic acid ester of a $C_8$-$C_{30}$ alkyl or, as therein more particularly described, a hydrocarbyl monoether of polyethylene glycol, (3) a $C_1$-$C_4$ alkyl acrylate or methacrylate, and, optionally, (4) a small amount of a polyethylenically unsaturated monomer.

U.S. Patent 7,649,047 and U.S. Patent 7,288,616, both to Tamareselvy et al., disclose multi-purpose alkali-swellable and alkali-soluble associative polymers. They are formed by polymerizing a monomer mixture of at least one acidic vinyl monomer, at least one nonionic vinyl monomer, a first associative monomer having a first hydrophobic end group, a second associative monomer having a second hydrophobic end group, and a crosslinking or chain transfer agent. The compositions exemplified by the patent include pearlized shampoo that includes mica and titanium dioxide.

US Patent 7,378,479 to Tamareselvy et al. discloses polymers that are the polymerization product of a monomer mixture comprising at least one amine-substituted vinyl monomer; at least one nonionic vinyl monomer; at least one associative vinyl monomer; at least one semihydrophobic vinyl surfactant monomer; and, optionally, one or more of hydroxyl-substituted nonionic vinyl monomer, crosslinking monomer, chain transfer agent or polymeric stabilizers. The polymers are said to be useful in, among other products, personal and healthcare products, and to have application, among other uses, as rheology modifiers, dispersants, and stabilizers. The patent exemplifies a conditioning shampoo that includes mica and titanium dioxide.

US Patent 7,217,752 (Schmucker-Castner et al.) discloses aqueous compositions containing a cross-linked alkali-swellable acrylate copolymer rheology modifier, a surfactant, an alkaline material, and substantially insoluble materials requiring suspension or stabilization including, for example, pearlescent material such as titanium dioxide coated mica, iron oxide coated mica, ethylene glycol mono-stearate, ethylene glycol distearate, bismuth oxychloride coated mica, myristyl myristate, guanine, glitter (polyester or metallic), and mixtures thereof. The polymers therein described include one or more carboxylic acid monomers having a total of from about 3 to about 10 carbon atoms (for example, acrylic acid, methacrylic acid, itaconic acid, fumaric acid, crotonic acid, maleic acid or aconitic acid, as well as half ester of polyacids such as maleic acid, fumaric acid, itaconic acid, or aconitic acid and the like with $C_{1-4}$ alkanols); one or more non-acid vinyl monomers as therein more particularly described, including various acylate or hydroxyacrylate esters wherein the ester portion has from 1 to 10 carbon atoms; and one or more polyunsaturated compounds carrying a reactive group capable of causing the copolymer to be cross-linked. The patent includes examples of shampoos and body washes that include pearlescent material.

WO 2010/026097 A1 (Graham et al.) describes rheology modifiers for use in home and personal care compositions. These modifiers are formed from four monomers. They include an amino-substituted vinyl monomer, a hydrophobic nonionic vinyl monomer (such as a $C_1$-$C_{30}$ alkyl ester of acrylic or methacrylic acid), an associative-like monomer (with a polyoxyalkylene unit endcapped with a hydrophobic group), and a further associative-like vinyl monomer. Graham et al. discloses that the copolymers can be used in a variety of personal care and topical health care products including hair care, cleansing products (for example, shampoos, body cleansers, body washes, and shower gels). Graham et al. further discloses that the copolymers therein described are "particularly useful as suspending agents for particulates, such as mica, pearlizing agents, beads and the like, microabrasives, and abrasives such as shower gels, masks and skin cleansers containing exfoliative scrub agents. Further personal care compositions based on structured liquids are disclosed in WO2007/090759. In the area of cleansing compositions in the form of structured liquids that include reflective particles, there remains a need for compositions in which the reflective character of such particles is optimized in a composition that affords desirable rheological properties and stability.

## SUMMARY OF THE INVENTION

The present composition relates to cleansing compositions that comprise:

i) a copolymer that is the polymerization product of:

a) from 0.1 to 5 wt. % of a first ethylenically unsaturated monomer selected from:

a diacid of the formula:

$$HOOC\text{-}CR^1\text{=}CR^2\text{-}COOH \qquad (I),$$

a cyclic anhydride precursor of diacid (I), the anhydride having the formula:

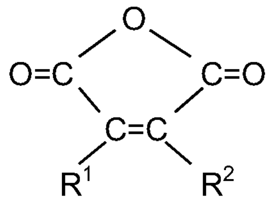

(II)

and combinations thereof,

wherein $R^1$ and $R^2$ are individually selected from H, $C_1$-$C_3$ alkyl, phenyl, chlorine and bromine,

b) from 15 to 60 wt. % of a second ethylenically unsaturated monomer selected from acrylic acid, methacrylic acid and combinations thereof,

c) from 30 to 75 wt. % of a (meth)acrylate monomer selected from $C_1$ to $C_8$ alkyl esters of (meth)acrylic acid and combinations thereof, and preferably selected from $C_1$ to $C_4$ alkyl esters of (meth)acrylic acid and combinations thereof,

d) from 1 to 25wt.% of an associative monomer of the formula:

$$R^4\text{-}CH\text{=}C(R^3)\text{-}C(O)\text{-}O\text{-}(R^5O)_a\text{-}R^6 \qquad (III)$$

wherein:

$R^3$ and $R^4$ are independently selected from H and $C_{1\text{-}3}$ alkyl,

each $R^5O$ is independently an oxyalkylene unit having from 2 to 4, preferably from 2 to 3 carbon atoms,

$R^6$ is selected from:

linear and branched alkyl having from 8 to 40, preferably from 8 to 30, more preferably from 10 to 22 carbon atoms, and

alkaryl, the alkyl group of which has from 8 to 40, preferably from 8 to 30, more preferably from 10 to 22 carbon atoms, such alkyl group being linear or branched, the alkaryl preferably being alkylphenyl; and

a is from 6 to 40;

wherein the copolymer is at least partially deprotonated. Preferably, the copolymer is sufficiently neutralized that it becomes substantially, completely deprotonated; preferably, the copolymer should be neutralized at least 20%, more preferably at least 30%, more preferably at least 50%, more preferably at least 70%; the greater the degree of neturalization, the closer it approaches being "substantially completely deprotonated" which we define to mean achieving at least 90% maximum viscosity at the concentration in which it is present; when "fully neutralized", the copolymer will have achieved maximum acceptable viscosity at the concentration in which it is present;

ii) cleansing surfactant;

iii) reflective particles; and

iv) water.

BRIEF DESCRIPTION OF THE FIGURES

Figures 1, 2 and 3 are photographs of various mica-containing shampoo compositions as described in the Examples.

DETAILED DESCRIPTION OF THE INVENTION

It has been found that the optical properties of reflective particles can be optimized in water-containing compositions that include one or more cleansing surfactants (e.g., shampoos, body washes, shower gels, liquid hand soaps, and the like) through the use of particular rheology modifying associative polymers. Without wishing to be bound by theory, it is believed that such polymers promote the formation of a structured liquid in which the reflective particles are dispersed in a manner that optimizes their ability to reflect light. In one or more embodiments, the subject compositions have been found to have a shiny or almost "metallic" appearance. It has further been found that the use of such polymers allows desirable visual properties to be achieved using relatively low levels of light-reflecting particles, in some embodiments at levels of not more than 0.2 wt. % of reflective particles, based on the total composition weight.

Additionally, the compositions of the subject invention have been found to be stable to phase separation over a relatively broad range of temperatures, providing, in one or more embodiments, stability at temperatures up to 45°C or greater, more particularly, at temperatures of from 5°C to 45°C, for periods of 8 weeks or more. The combination of stability and optical benefits offers the potential of creating numerous visual effects; for example, layering or otherwise combining compositions with different visual characteristics, to create products with stripes, swirls and other patterns. Moreover, in one or more embodiments, the subject polymers provide both desirable optical benefits and desirable stability benefits at relatively low reflective particle levels.

In one or more embodiments, the subject compositions have both a brightness value of at least 70 and a total specular reflectance value of at least 130, preferably at least 140, more preferably at least 150, as determined by the spectrophotometric procedure set forth below, and preferably contain reflective particles in an amount up to 0.2wt.%, more preferably up to 0.15wt.%, based on the total weight of the composition.

Copolymer

The copolymers of the invention are the polymerization product, more particularly, the addition polymerization product, of first ethylenically unsaturated monomer, second ethylenically unsaturated monomer, (meth)acrylate monomer and associative monomer as described above. The copolymers are generally random in structure; preferably the copolymers are linear. Throughout this specification, unless otherwise indicated, the wt. % of monomer in the reported monomer ranges, levels and ratios is based on the total weight of the starting monomers, that is to say, based on total monomer added. Unsaturated monomers will lose unsaturation as they are polymerized and may further transform into salts when neutralised. Monomer nomenclature and levels, ranges and ratios are made with reference to the unsaturated and, where appropriate, unneutralised starting monomer materials.

In one or more embodiments, it is contemplated that the starting monomers are incorporated into the copolymer in amounts generally reflective of the amounts in which they are added as starting materials; in such embodiments it is contemplated that the weight percentages and ratios of such monomers may alternatively be reported as being based on the total weight of the resulting copolymer. In other embodiments it is contemplated that the starting monomers are incorporated into the copolymer in amounts within 10 % by weight and, more particularly, in amounts within 5 % by weight of the amount in which they are added.

In one or more embodiments the ratio, by weight, of first ethylenically unsaturated monomer (a) to the associative monomer (d) is preferably from 1:3 to 1:30; more preferably from 1:5 to 1:25 and, even more preferably from 1:5 to 1:22. In one or more embodiments, a ratio of first ethylenically unsaturated monomer (a) to associative monomer (d) of from 1:10 to 1:25, more particularly, from 1:15 to 1:22 is of particular interest. These weight ratios are particularly advantageous with the preferred first ethylenically unsaturated monomers and associative monomers listed below.

First Ethylenically Unsaturated Monomer

As noted above, the first ethylenically unsaturated monomer is selected from selected from:

a diacid of the formula:

$$HOOC\text{-}CR^1\text{=}CR^2\text{-}COOH \qquad (I),$$

a cyclic anhydride precursor of diacid (I), the anhydride having the formula:

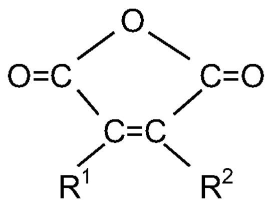

(II)

and combinations thereof,

wherein $R^1$ and $R^2$ are individually selected from H, $C_1$-$C_3$ alkyl, phenyl, chlorine and bromine and, in one or more embodiments, are preferably individually selected from H and $C_1$-$C_3$ alkyl. In one or more embodiments of particular interest the first ethylenically unsaturated monomer is selected from maleic acid, fumaric acid, citraconic acid, maleic anhydride, citraconic anhydride, and combinations thereof, with first ethylenically unsaturated monomer selected from maleic and citraconic acid, and maleic and citraconic anhydride being of particular interest. In other preferred embodiments the first ethylenically unsaturated monomer is selected from maleic acid and maleic anhydride, with maleic anhydride being of particular interest. While fumaric acid is contemplated for use herein, in one or more embodiments, it is preferred that fumaric acid absent from the subject copolymers or is present in only minor amounts, i.e., less than 0.05 wt. %, preferably less than 0.005 wt. %, based on the total weight of monomer charged.

The first ethylenically unsaturated monomer may be employed in amounts of from 0.1 to 5 wt. %, preferably from 0.2 to about 4 wt. %, more preferably from 0.2 to 2 wt. %, and, in one or more embodiments, from 0.3 to 0.6 wt. %, based on the total weight of monomer charged. In one or more embodiments of particular interest, maleic anhydride or maleic acid provides at least 50 % by weight, more preferably at least 80 % by weight, even more preferably, at least 95 % by weight, of the total weight of the first ethylenically unsaturated monomer. In the examples, it is seen that whether the starting unsaturated monomer is predominantly anhydride (e.g., maleic anhydrie of polymer A as used, for example, in Examples 3, 5, 6 and 7) or predominantly acid (e.g., maleic acid of polymer D as used, for example, in Exmaples 14 and 15), results are superior relative to Example 2, for example, where copolymer is Aculyn® 28.

Second Ethylenically Unsaturated Monomer

The second ethylenically unsaturated monomer is selected from acrylic acid, methacrylic acid and combinations thereof. The second ethylenically unsaturated monomer may be employed in amounts of from 15 to 60 wt.%, preferably from 20 to 55 wt. %, more preferably from 30 to 50 wt.%, based on total monomer charged. In one or more embodiments of interest the amount of second ethylenically unsaturated monomer is from 25 to 40 wt.%, based on total monomer charged; in one or more other embodiments of interest, the amount of second ethylenically unsaturated monomer is from 20 to 40 wt. % based on total monomer charged. In one or more embodiments of particular interest, methacrylic acid provides at least 50 % by weight, more preferably at least 70 % by weight, even more preferably, at least 90 % by weight, of the total weight of the second ethylenically unsaturated monomer.

(Meth)acrvlate Monomer

The (meth)acrylate monomer may be selected from $C_1$ to $C_8$ alkyl esters of acrylic acid and $C_1$ to $C_8$ alkyl esters of methacrylic acid and combinations thereof, with $C_1$ to $C_4$ alkyl esters of such acids being of particular interest. Illustrative ester monomers are ethyl acrylate, methyl acrylate, ethyl methacrylate, methyl methacrylate, butyl acrylate, and butyl methacrylate. The (meth)acrylate monomer may be employed in amounts of from 30 to 75 wt.%, preferably from 40 to 75wt. %, more preferably from 45 to 70 wt.%, based on total monomer charged. In one or more embodiments of particular interest, $C_1$ to $C_4$ alkyl esters of acrylic acid, preferably ethyl acrylate, provide at least 50 % by weight, more preferably at least 70 % by weight, even more preferably, at least 90 % by weight, of the total weight of the (meth)acrylate monomer.

Associative Monomer

a) The associative monomer, also termed "surfmer", has the formula:

$$R^4\text{-}CH{=}C(R^3)\text{-}C(O)\text{-}O\text{-}(R^5O)_a\text{-}R^6 \qquad (III)$$

wherein:

$R^3$ and $R^4$ are independently selected from H and $C_{1-3}$ alkyl,

each $R^5O$ is independently an oxyalkylene unit having from 2 to 4, preferably from 2 to 3 carbon atoms,

$R^6$ is selected from:

linear and branched alkyl having from 8 to 40, preferably from 8 to 30, more preferably from 10 to 22 carbon atoms, and

alkaryl, the alkyl group of which has from 8 to 40, preferably from 8 to 30, more preferably from 10 to 22 carbon atoms, such alkyl group being linear or branched, said alkaryl preferably being alkylphenyl; and

a has a value of from 6 to 40, preferably from 15 to 35, most preferably from 20 to 30.

Of particular Interest in one or more embodiments is an associative monomer of the formula:

$$CH_3[CH_2]_b\text{-}CH_2\text{-}[OCH_2CH_2]_a\text{-}O\text{-}C(O)C(R^3)\text{=}CH(R^4) \qquad IV$$

in which $R^3$, $R^4$ and a are as described above, and b has a value of from 6 to 38, preferably from 6 to 28, and more preferably from 8 to 20.

In the Formula III and Formula IV monomers, $R^3$ is preferably a methyl group and $R^4$ is preferably H. In the above described associative monomers, a and b represent the number of their respective oxyalkylene and $-CH_2-$ repeat units and generally are integers. In one or more embodiments of interest a is greater than or equal to b.

The associative monomer may be employed in amounts of from 1 to about 25 wt. %, preferably from 2 to 20 wt. %, and more preferably from 2 to 15 wt. %, based on total monomer added. In one or more embodiments of particular interest the amount of associative monomer employed is from 5 to 12 wt. %, based on total monomer added.

Unless otherwise indicated, reference to the aforesaid monomers includes all reactive forms thereof. In one or more embodiments, it is contemplated that one or more acid groups of the ethylenically unsaturated starting monomers may be neutralized to salt form. Typical salt counter-ions to the acid groups are alkali metals, especially sodium and potassium, and ammonium and triethanolammonium cations.

Minor amounts of additional monomers may be employed, provided that they do not undesirably impact the swelling ability of the resultant copolymer. If present, the total amount of additional monomers is preferably less than 5wt. %, more preferably less than 2 wt. % of the total monomer charge. In one or more embodiments, the subject invention contemplates the use of cross-linking monomer as an additional optional monomer. When present, cross-linking monomer is employed in an amount up to 1 wt.%, more particularly an amount up to 0.5 wt.%, based on total monomer charge. In other more preferred embodiments, the additional monomer does not include cross-linking monomer, i.e., the copolymer is not cross-linked. Preferably the copolymer is neutralized (e.g., by the addition of NaOH or other alkaline agent which deprotonates the copolymers) prior to combining with surfactant and the polymer is not cross-linked. This helps ensure that the copolymer expands and structures. Preferably the copolymer is both linear and not cross-linked. Preferably, such linear, non-cross-linked copolymer is neutralized before combining with surfactant as noted above.

In one or more embodiments of interest the copolymers are the polymerization product of monomers that consist essentially of monomers (a) to (d) and, in one embodiment of particular interest the copolymers are the polymerization product of monomers that "consist of" monomers (a) to (d). In one or more embodiments, it is desirable that itaconic acid is absent from the subject copolymers or is present in only minor amounts, i.e., less than 0.05 wt.%, preferably less than 0.005 wt.%, based on the total weight of monomer charged.

Copolymers as described herein, can be prepared according to the procedures described in PCT Application No. PCT/EP2012/068732, entitled Thickening Vinyl Copolymers, filed September 24, 2012, incorporated herein by reference. In general, such copolymers are prepared by addition polymerization reactions. Such polymerization is typically carried out in an aqueous medium and typically employs an initatior, for example, ammonium persulfate, potassium persulfate, water soluble azo initiators, for example, 2,2'-azobis(2-amidinopropane) hydrochloride, and the like, or a redox system such as FE(II) and cumenehydroperoxide or dimethylhydroperoxide. Reaction temperatures of choice will depend, in part, on the selection of initiator, for example, when ammonium persulfate is employed, reaction temperatures are typically in the range of 80 to 95°C, while lower temperatures may be more appropriate for a redox system. Reaction pH also depends in part on the selection of initiator; with pH values below 3, more particularly from 2.5 to 3 being of interest in many reactions.

The polymerization may be carried out in the presence of surfactant. Without wishing to be bound to theory, the surfactant may act as an electrostatic stabilizer, however, the amount of surfactant present should not give rise to

undesirable coagulation. In one or more embodiments it is desirable that surfactant is present in the polymerization medium, in an amount of less than 0.5wt.%, based on the total weight of all components thereof. If desired, the copolymer formed may, but need not be isolated from the resulting dispersion. Isolation of the copolymer may be achieved following conventional separation and work-up techniques.

While copolymers of lesser molecular weight are contemplated, in one or more embodiments it is preferred that the copolymers have a weight average molecular weight of at least 500,000 g $mol^{-1}$. Preferably the copolymer is not crosslinked.

The copolymers require alkaline conditions to swell. Swelling, also referred to neutralization or deprotonation, is typically carried out with a neutralizing agent that is preferably a base such as an amine base or an alkali metal hydroxide, most preferably sodium hydroxide. While the copolymers may be swelled prior to being incorporated into the subject cleansing compositions, they are more typically added in an unswollen state and swelled in the course of preparing the cleansing compositions of interest. The method of cleansing composition manufacture should not, however, interfere with attaining the desired degree of copolymer swelling. For example, it is generally preferable that the copolymer be treated with neutralizing agent prior to the composition being formed into a structured liquid, e.g. prior to micelle formation, as surfactant molecules and micellar structures can interfere with polymer swelling. As noted above, this is also consistent with use of linear, non-cross-linked copolymer. In one or more embodiments, it is preferred that copolymer (i) is at least partially neutralized to achieve a considerable viscosity increase at a given concentration prior to incorporation of cleansing surfactant in an amount sufficient to structure the composition. In one or more embodiments it is preferred that copolymer (i) is fully neutralized, i.e., that it achieves the maximum available viscosity at the concentration in which it is present. As used herein, the term "deprotonated" is alternately applied to denote a copolymer that has been neutralized. Reference to copolymer (i) being "substantially completely deprotonated" means that the copolymer achieves at least 90% of its maximum available viscosity at the concentration in which it is present. Fully neutralized, as noted, indicates maximum achievable viscosity at concentration at which copolymer is present. While it is preferred that the copolymer be substantially completely deprotonated (achieve at least 90% maximum available viscosity at concentration present as noted), depending on degree of neutralization (which may vary, for example, by concentration, order of addition),the copolymer may be 30% neutralized, 40% neutralized, 50% neutralized and so forth. Preferably, it is at least 20% neutralized, more preferably at least 30% neutralized, more preferably at least 50% neutralized, more preferably at least 70% neutralized; more preferably there should be sufficient neutralization such that copolymer is substantially completely deprotonated (90% maximum viscosity); even more preferably it is sufficiently neutralized to be "fully" neutralized (maximum available viscosity achieved).

Cleansing Surfactant

Compositions of the invention comprise one or more cleansing surfactants that are cosmetically acceptable and suitable for topical application to the hair and/or skin. Further surfactants may be present as emulsifiers. Suitable cleansing surfactants are selected from anionic, non-ionic, amphoteric and zwitterionic surfactants and mixtures thereof. Examples of such surfactants are provided below.

Anionic Cleansing Surfactant

Among the suitable anionic cleansing surfactants are the alkyl sulphates, alkyl ether sulphates, alkaryl sulphonates, alkanoyl isethionates, alkanoyl sarcosinates, alkanoyl glycinates, alkyl succinates, alkyl sulphosuccinates, alkyl ether sulphosuccinates, alkyl sarcosinates (particularly N-alkyl sarcosinates), alkyl phosphates, alkyl ether phosphates, and alkyl ether carboxylic acids and salts thereof, especially their sodium, potassium, magnesium, ammonium and mono-, di- and triethanolamine salts. The alkyl and alkanoyl (aka acyl) groups generally contain from 8 to 20, preferably from 10 to 16, carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule. Examples thereof are sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryol isethionate, lauryl ether carboxylic acid, sodium N-lauryl sarcosinate, sodium cocoyl glycinate, sodium stearoyl glycinate, sodium lauroyl glycinate, sodium lauroyl sarcosinate, and potassium cocoyl glycinate.

In one or more embodiments, preferred anionic surfactants are the salts of alkyl sulfates and alkyl ether sulfates. These materials have the respective formulae $R^8OSO_3M$ and $R^7O(C_2H_4O)_xSO_3M$, wherein $R^7$ and $R^8$ are preferably saturated or unsaturated alkyl of from 8 to 20 carbon atoms, x is an integer having a value of from about 1 to about 10, and M is a cation such as ammonium, alkanolamines, such as triethanolamine, monovalent metal, such as sodium and potassium, and polyvalent metal cation, such as magnesium, and calcium. Most preferably $R^7$ and $R^8$ have 10 to 16 carbon atoms, in a linear rather than branched chain.

In one or more embodiments preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3), with sodium lauryl ether sulphate (n)EO where n=1 being of particular interest.

In other embodiments, particularly where very mild compositions are desired, it is desirable that such compositions have 3 % or less, preferably 2 wt. % or less, more preferably 1 wt. % or less of alkyl/alkyl ether sulphate anionics (e.g., sodium dodecyl sulphate, sodium lauryl sulfate, sodium lauryl ether sulfate, and other anionic species of alkyl- and alkyl ether sulphates). In one or more embodiments, no (i.e., none whatsoever except for possible impurities) or substantially no (e.g., 0.2 wt. % or less) of alkyl/alkyl ether sulphate anionics are present in the subject compositions.

In one or more applications, it is preferred that the anionic surfactant is selected from one or more of alkanoyl isethionate, alkanoyl sarcosinate, alkanoyl glycinate, and salts thereof, especially their potassium and, more particularly, sodium salts, with mixtures that include alkanoyl glycinate and/or salts thereof being of particular interest.

Depending upon the desired end use, the total amount of anionic cleansing surfactant is from 0 to 25 wt. %, more preferably from 3 wt. % to 18 wt. %, most preferably from 6 wt. % to 15 wt. %, of the total composition. For many compositions, it is desirable that at least a portion of the cleansing surfactant is anionic surfactant.

Nonionic Cleansing Surfactant

Compositions of the invention may contain non-ionic cleansing surfactant. In one or more embodiments non-ionic cleansing surfactants is present in an amount within the range of 0 to 5 wt. %.

Nonionic surfactants that can be included in compositions of the invention include condensation products of aliphatic ($C_8$ - $C_{18}$) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula:

$$R^9\text{-}(OCH_2CH_2)_nOH,$$

where $R^9$ is an alkyl chain of C12 to C15, and n is 5 to 9.

Other suitable nonionic surfactants include mono- or di-alkyl alkanolamides. Examples include coco mono- or di-ethanolamide and coco mono-isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

$$R^{10}O\text{-}(G)_z$$

wherein $R^{10}$ is a branched or straight chain alkyl group which may be saturated or unsaturated and G is a saccharide group. $R^{10}$ may represent a mean alkyl chain length of from about $C_5$ to about $C_{20}$. Preferably $R^{10}$ represents a mean alkyl chain length of from about $C_8$ to about $C_{12}$. Most preferably the value of $R^8$ lies between about 9.5 and about 10.5. G may be selected from $C_5$ or $C_6$ monosaccharide residues, and is preferably a glucoside. G may be selected from the group comprising glucose, xylose, lactose, fructose, mannose and derivatives thereof. Preferably G is glucose.

The degree of polymerisation, z, may have a value of from about 1 to about 10 or more. Preferably, the value of z lies from about 1.1 to about 2. Most preferably the value of z lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic cleansing surfactants which can be included in compositions of the invention include the $C_{10}$-$C_{18}$ N-alkyl ($C_1$-$C_6$) polyhydroxy fatty acid amides, such as the $C_{12}$-$C_{18}$ N-methyl glucamides, as described for example in WO 92 06154 and US 5 194 639, and the N-alkoxy polyhydroxy fatty acid amides, such as $C_{10}$-$C_{18}$ N-(3-methoxypropyl) glucamide.

Amphoteric/zwitterionic Surfactant

Amphoteric or zwitterionic cleansing surfactant can be included in an amount ranging from 0 wt. % to about 8 wt. %, preferably from 0.5 wt. % to 6 wt. %, and more preferably from 1 wt. % to 4 wt. % of the total composition. The inclusion of such surfactant aids in foaming or lathering.

Examples of such amphoteric or zwitterionic cleansing surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates acyl glutamates, and salts thereof, wherein the alkyl and acyl groups have from 8 to 19 carbon atoms. Typical amphoteric and zwitterionic

surfactants for use in compositions of the invention include lauryl amine oxide, cocodimethyl sulphopropyl betaine, lauryl betaine, cocamidopropyl betaine, cocoamphoacetate, and salts thereof, with cocamidopropyl betaine and salts thereof being of particularly interest in one or more embodiments.

Mixtures of any of the foregoing amphoteric or zwitterionic surfactants may also be suitable. In one or more embodiments, preferred mixtures are those of cocamidopropyl betaine with further amphoteric or zwitterionic surfactants as described above. A preferred further amphoteric or zwitterionic surfactant is sodium cocoamphoacetate.

In one or more embodiments the cleansing surfactant is a surfactant system comprising anionic surfactant in combination with amphoteric and/or zwitterionic surfactant, with combinations of salts one or more alkyl/alkyl ether sulfates of the formula $R^8OSO_3M$ or $R^7O(C_2H_4O)_xSO_3M$ as described above.

In other embodiments of interest the cleansing surfactant is a mild surfactant system comprising:

a) an alkanoyl surfactant selected from one or more of alkanoyl glycinate, alkanoyl sarcosinate, and salts thereof, wherein the alkyl group on the alkanoyl chain is $C_8$ to $C_{20}$, preferably $C_{12}$ to $C_{16}$ straight chain alkyl (e.g., lauroyl, cocoyl or myristoyl glycinate);

b) a fatty acyl isethionate product which product comprises 40 to 80 % (of the product) of one or more of fatty acyl isethionate and salts thereof and 15 to 50 % (of the product) of free fatty acid and/or fatty acid salt/soap (the product may also comprise isethionate salts, typically present at less than 5 % of the product and may further comprise traces, i.e., typically less than 2 % of product, or other impurities).

A mild surfactant system of this type is described in further detail in US 8,268,767 incorporated herein by reference.

The total amount of cleansing surfactant in the compositions of the invention generally ranges from 3 to 35 percent by weight, preferably from 5 to 25 percent by weight, based on the total weight of the composition, with compositions containing cleansing surfactant in an amount of from 5 to 15 percent by weight, based on the total weight of the composition, in one or more embodiments.

## Light Reflective Particles

A variety of light reflecting particles may be employed in the subject compositions. As used herein "light reflecting" or "reflective" in reference to the particles means that particles give the product a pearly, shiny and/or shimmering appearance due to their light reflecting ability. Preferably, the light reflective particles are in solid form (as opposed to being liquids or gases) at temperature of 25°C and 1 atmosphere of pressure, and desirably remain in solid form in the course of composition manufacture.

Illustrative but not limiting examples of light reflecting particles are glitter, optically reflective polymeric flakes and beads, bismuth oxychloride (including, for example, single crystal platelets), mica, crystallized surfactants (such as, for example, ethylene glycol distearate), and pearlized waxes, as well as particles where the reflective character is provided by a coating. Suitable bismuth oxychloride crystals are available from EM Industries, Inc. under the trademarks Biron® NLY-L-2X CO and Biron® Silver CO (wherein the platelets are dispersed in castor oil); Biron® Liquid Silver (wherein the particles are dispersed in a stearate ester); and Nailsyn® IGO, Nailsyn® II C2X and Nailsyn® II Platinum 25 (wherein the platelets are dispersed in nitrocellulose). Most preferred is a system where bismuth oxychloride is dispersed in a $C_{20}$-$C_{40}$ alkyl ester such as in Biron® Liquid Silver.

Suitable micas include titanium dioxide coated mica, such as, for example coated platelet materials available from EM Industries, Inc. These include Timiron® MP-10 (micrometer particle size range 10-30 μm), Timiron® MP-14 (particle size range 5-30 μm), Timiron® MP-30 (particle size range 2-20 μm), Timiron® MP-101 (particle size range 5-45 μm), Timiron® MP-111 (particle size range 5-40 μm), Timiron® MP-1001 (particle size range 5-20 μm), Timiron® MP-155 (particle size range 10-40 μm), Timiron® MP-175 (particle size range 10-40 μm), Timiron® MP-115 (particle size range 10-40 μm), and Timiron® MP-127 (particle size range 10-40 μm). Most preferred is Timiron® MP-111. The weight ratio of titanium dioxide coating to the mica platelet may range from about 1:10 to about 5:1, preferably from about 1:1 to about 1:6, more preferably from about 1:3 to about 1:4 by weight. In one or more embodiments, the only titanium dioxide present in the subject compositions is titanium dioxide present as a coating on mica or other reflective particles.

Suitable coatings for mica other than titanium dioxide may also achieve the appropriate optical properties required for the present invention. Non-coated micas are also suitable for use herein.

Pearalized waxes include products available from Cognis under the trademark Euperlan®, which waxes are available in surfactant concentrates.

Still other light reflective particles suitable for use herein are materials known as interference pigments. Interference pigments are materials having a substrate on which layers of refractive material are deposited. Light waves are

reflected, refracted and scattered by the layers giving rise to wave interferences that, in turn, affect color perception. The number and thickness of such layers, the choice of materials from which the layers are formed (including the refractive properties thereof), and the choice and geometry of substrate materials are among the factors that impact the properties of interference pigments.

Among the materials of interest as interference pigment substrates are natural mica, synthetic mica, aluminum oxide, silica, calcium aluminum oxide, barium sulfate, calcium aluminum borosilicate, and bismuth oxychoride, with mica, silica, and aluminum oxide being among the substrate materials of preference and, in the case of silica and aluminum oxide, particularly the flake forms thereof. In one or more embodiments the substrate is most preferably mica.

Materials suitable for use as layer materials include, for example, $TiO_2$, $Fe_2O_3$, ZnO, ZnS, SnO, $Al_2O_3$, and $Cr_2O_3$ with $TiO_2$ being of particular interest in one or more embodiments. Optionally, the interference pigment may include absorption pigment which may, but need not, form part of the layer structure.

Interference pigments suitable for use herein are available from numerous suppliers including Merck, EMD Chemicals, Inc., BASF and Engelhard.

The amount of the light reflecting particles may range from 0.05 to 5 wt. % of the total composition, depending upon the particles employed and the visual effect desired, with light reflective particle ranges of from 0.1 to 2 wt. % of the total composition generally being preferred for many applications. In one or more embodiments of interest, particularly in the case of mica, it is preferred that the amount of the light reflecting particles is from 0.05 to 1 wt. %, more particularly from 0.05 to 0.5 wt. %, and even more particularly from 0.1 to 0.4 wt. % of the total composition. In one or more embodiments of interest desirable optical properties are achieved employing light reflective particles in an amount of 0.05 to 0.3 wt. %, preferably in an amount of 0.05 to 0.2 wt. %, based on the total weight of the composition.

Water

The subject compositions are aqueous compositions, typically existing as structured liquids in the form of aqueous dispersions or emulsions, particularly oil-in water emulsions. Water typically accounts for from 40 to 95 wt. %, preferably from 50 to 90 wt. %, of the composition, based on the total weight thereof. Compositions containing water in an amount of 60 to 85 wt. %, based on total composition weight are of particular interest. Water is preferably present in deionized form.

Additional Optional Components

Salt

Salt can assist in building the viscosity of a structured liquid. In micellar liquids, salt helps promote micelle formation. Cosmetically acceptable organic or inorganic salts may be employed herein, so long as the salts are sufficiently soluble in the structured liquid and do not interact with other composition components to give insoluble species or undesirable complexes. Monovalent salts are generally less prone to form complexes and are preferred in one or more embodiments. Alkali metal salts, especially sodium and potassium salts are of particular interest, with alkali metal citrates and acetates, and chlorides being among the salts of particular interest, with sodium chloride and potassium chloride being preferred in one or more embodiments.

When present, the salts are often added toward the later stages of composition preparation to adjust viscosity to a desired final value.

Silicones

The compositions of the invention optionally contain one or more silicones. Among the silicones suitable for use herein are silicones oils, gums and elastomers employed in liquid cleansing compositions for hair and/or skin, which materials are frequently available as pre-formed emulsions. The viscosity of the emulsified silicone, as distinguished from the cleansing compositions in which it is employed, is typically at least 10,000 cSt, and preferably is at least 60,000 cSt, with viscosities of at least 500,000 cSt, more particularly at least 1,000,000 cSt being of interest in one or more embodiments. Preferably the viscosity does not exceed $10^9$ cSt for ease of formulation.

Suitable silicone oils include polydiorganosiloxanes, in particular polydimethylsiloxanes which have the CTFA designation dimethicone. Also suitable for use in compositions of the invention are polydimethyl siloxanes having hydroxyl end groups, which have the CTFA designation dimethiconol. Also suitable for use in compositions of the invention are silicone gums having a slight degree of cross-linking, as are described for example in WO 96/31188.

Many of the emulsified silicones for use in the compositions of this invention, will have an average silicone droplet size in the composition of less than 30, preferably less than 20, more preferably less than 10 micrometers, ideally from 0.01 to 1 micrometer.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions/ microemulsions of dimethiconol. Cross-linked silicone gums are also available in a pre-emulsified form, which is advantageous for ease of formulation.

A further preferred class of silicones for inclusion in the compositions of this invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt. Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 cationic emulsion and the non-ionic emulsions DC2-7224, DC2-8467, DC2-8177 and DC2-8154 (all ex Dow Corning).

Combinations of amino and non amino functional silicones may also be used.

In many compositions total amount of silicone is preferably from 0.01 wt. % to 10 wt. % of the total composition more preferably from 0.1 wt. % to 5 wt. %, most preferably 0.5 wt. % to 3 wt. %.

Emollients/Fatty Materials

The compositions may further include, for example, emollients, fatty acids, fatty alcohols, and combinations thereof.

Emollient materials may be in the form of natural or synthetic esters and hydrocarbons. Amounts of the emollients may range anywhere from 0.1 (or less) to 40%, preferably between 1 and 30 % by weight of the composition, depending upon the product form of interest and choice of emollient materials.

Among the ester emollients are:

Alkyl esters of saturated fatty acids having 10 to 24 carbon atoms. Examples thereof include behenyl neopentanoate, isononyl isonanonoate, isopropyl myristate and octyl stearate.

Ether-esters such as fatty acid esters of ethoxylated saturated fatty alcohols.

Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of $C_1$-$C_{30}$ alcohols.

Wax esters such as beeswax, spermaceti wax and tribehenin wax.

Sugar ester of fatty acids such as sucrose polybehenate and sucrose polycottonseedate.

Natural ester emollients principally are based upon mono-, di- and triglycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives.

Hydrocarbons which are suitable cosmetically acceptable carriers include petrolatum, mineral oil, $C_{11}$-$C_{13}$ isoparaffins, polybutenes, and especially isohexadecane, available commercially as Permethyl® 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids.

Humectants may also be employed. These are generally polyhydric alcohol-type materials. Typical polyhydric alcohols include propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of adjunct humectant may range anywhere from 0.5 to 20 %, preferably between 1 and 10 %, most preferably between 2 and 8 % by weight of the composition.

Adjunct Thickeners

A variety of other thickening agents may be included alongside the thickening copolymers of the present invention. Representative classes are:

a. Taurate Polymers

Examples include hydroxyethyl acrylate/ sodium acryloyldimethyl taurate (e.g. Simulgel® NS and INS 100), acrylate/ sodium acryloyldimethyl taurate (e.g. Simulgel® EG), sodium acryloyldimethyl taurate (e.g. Simulgel® 800) and ammonium acryloyldimethyl taurate/ vinyl pyrrolidone (e.g. Aristoflex® AVC).

b. Polyacrylamide Polymers

The compositions can optionally comprise vinyl polymerized polyacrylamide polymers, especially nonionic polyacrylamide polymers including substituted branched or unbranched polymers. Preferred among these polyacrylamide polymers is the nonionic polymer given the CTFA designation polyacrylamide and isoparaffin and laureth-7, available under the tradename Sepigel® 305 from Seppic Corporation.

Other polyacrylamide polymers useful herein include multi-block copolymers of acrylamides and substituted acrylamides with acrylic acids and substituted acrylic acids. Commercially available examples of these multi-block copolymers include Hypan® SR150H, SS500V, SS500W, SSSA100H, from Lipo Chemicals, Inc.

c. Polysaccharides

"Polysaccharides" refer to gelling agents that contain a backbone of repeating sugar (i.e., carbohydrate) units. Nonlimiting examples of polysaccharide gelling agents include those selected from the group consisting of cellulose, carboxymethyl hydroxyethylcellulose, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof.

d. Gums and Clays

Nonlimiting examples include materials selected from the group consisting of acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, laponite, bentonite, hyaluronic acid, hydrated silica, hydroxypropyl chitosan, hydroxypropyl guar, karaya gum, kelp, locust bean gum, natto gum, potassium carrageenan, propylene glycol alginate, sclerotium gum, sodium carboxymethyl dextran, sodium carrageenan, tragacanth gum, xanthan gum, and mixtures thereof.

Other

Water-soluble benefit agents for skin and/ or hair applications may optionally be formulated into the compositions. A variety of water-soluble skin benefit agents can be used and the level can be from 0.1 to 40 % but preferably from 1 to 30 % by weight of the composition, and, in one or more embodiments, from1 to 20% by weight of the composition. These materials include, but are not limited to, polyhydroxy alcohols. Preferred water soluble skin benefit agents are glycerin, sorbitol and polyethylene glycol.

Preservatives can desirably be incorporated into the personal care compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are preferably employed in amounts ranging from 0.0001 % to 2 % by weight of the composition.

Compositions of the present invention may include vitamins. Illustrative vitamins are Vitamin A (retinol), Vitamin $B_2$, Vitamin $B_3$ (niacinamide), Vitamin $B_6$, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001 to 10 %, preferably from 0.01 % to 1 %, optimally from 0.1 to 0.5 % by

weight of the composition.

Desquamation promoters may also be present. Illustrative are the alpha-hydroxycarboxylic acids and beta-hydroxycarboxylic acids. The term "acid" is meant to include not only the free acid but also salts and $C_1$-$C_{30}$ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from about 0.01 to about 15 % by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, chamomile, licorice, aloe vera, grape seed, citrus unshui, willow bark, sage, thyme, rhamose (Uncaria) and rosemary.

Also included may be such materials as lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Amounts of these materials may range from about 0.000001 to about 10 %, preferably from about 0.0001 to about 1 % by weight of the composition.

Colorants, opacifiers, and abrasives may also be included in compositions of the present invention. Each of these substances may range from about 0.05 to about 5 %, preferably between 0.1 and 3 % by weight of the composition.

Fragrance, whether encapsulated or as free fragrance, may also be employed in the subject compositions.

The compositions are preferably adjusted to final pH and viscosity following the addition of cleansing surfactant and other ingredients to neutralized copolymer (i). The compositions of the present invention are preferably in the form of structured liquids having final viscosities of from 3000 to 10000 cP, more preferably from 4000 to 6000cP (Brookfield Viscometer, 30°C, 20 rpm, spindle 5.

In one or more embodiments it is desirable that the compositions are transparent or translucent. By "transparent" it is meant that light passes through the material without appreciable scattering so that objects situated behind the material can be clearly seen, whereas "translucent" means that light passes through the material but is diffused such that objects behind the material are visible, but not distinct.

The compositions may be dispensed from any of a number of dispensers, with pumps, tubes and bottles and, for some applications, sachets being of particular interest.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above. In specifying any range of concentration or amount, any particular upper concentration or amount can be associated with any particular lower concentration or amount. Reported ranges are inclusive of their endpoints.

Except in the operating and comparative examples, or where otherwise explicitly indicated, all numbers in this description indicating amounts of material ought to be understood as modified by the word "about".

The following examples will more fully illustrate the embodiments of this invention. The Examples are not intended to limit the scope of the invention in any manner. All parts, percentages and proportions referred to herein and in the appended claims are by weight unless otherwise indicated.

EXAMPLE 1

ASSOCIATIVE POLYMER SYNTHESIS

Associative Monomer Synthesis

Associative monomer having the structure:

$$CH_3[CH_2]_b\text{-}CH_2\text{-}[OCH_2CH_2]_a\text{-}O\text{-}C(O)C(CH_3)\text{=}CH_2 \qquad V$$

wherein:

a = 23 (nominal) and

b = 12 (nominal),

was synthesized as follows:

Brij®-35 polyoxyethylene (23) lauryl ether (150 g) (Sigma Aldrich) was dissolved in 500 ml anhydrous dichloromethane under a nitrogen atmosphere and cooled in an ice bath to 5 °C. Triethylamine (18.6 g) was added via syringe before methacryloyl chloride (20.9 g) was added dropwise over a 30 minute period. After complete addition, the solution was allowed to warm to room temperature and the reaction stirred for 4 weeks. The solution was then filtered to remove the resulting precipitate and washed once with saturated sodium hydrogen carbonate solution (200 ml) and once with saturated brine (200 ml). The solution was then passed through a column containing basic alumina before the product was dried with anhydrous magnesium sulphate, filtered and the solvent removed in vacuo. In subsequent examples this product is referred to as Surfmer D1.

General Polymer Synthesis

A series of copolymers were prepared using Surfmer D1 and other materials identified in Table 1. The starting materials were charged, in the indicated amounts, to a round bottom flask. The mixture was sealed and purged with nitrogen for 60 minutes before adding deoxygenated water and sodium dodecyl sulfonate (SDS) and then stirring into a pre-emulsion. A multineck round bottom flask was fitted with a nitrogen sparge and overhead stirrer. Deoxygenated water and an aqueous solution of 30 wt% SDS were added to the multineck flask, stirred at 250 rpm and heated to 90°C to form a surfactant solution. An aqueous solution of ammonium persulfate (0.15 g active in 1 ml of water) was added to the surfactant solution via syringe. The pre-emulsion was fed into the surfactant solution via peristaltic pump over 150 minutes. After complete addition an additional aqueous solution of ammonium persulfate (0.15 g active in 1 ml of water) was added and the reaction stirred for a further 240 minutes, forming a milky dispersion of the polymer. Table 1 provides further details regarding the amounts of materials employed. In the Examples described in Table 2, the copolymers were added in the form of the final dispersions.

TABLE I

| | **Raw Material Charge (Weight** in **Grams)** | | | |
|---|---|---|---|---|
| **Component** | Polymer A | Polymer B | Polymer C | Polymer D |
| **Pre-emulsion** | | | | |
| Ethyl Acrylate | 66.8 | 75.3 | 78.0 | 61.6 |
| Methacrylic Acid | 37.7 | 45.9 | 46.2 | 34.8 |
| Maleic anhydride | 0.515 | 0.627 | - | - |
| Maleic acid | - | - | - | 0.563 |
| Surfmer D1 | 10.0 | 12.18 | 4.06 | 9.23 |
| Polybutylmethacryl | | - | 4.06 | - |
| ate Macromer[1] | | | | |
| Trimethylolpropane Triacrylate | | 0.670 | 0.670 | - |
| Water | 26.5 | 38.3 | 34.3 | 25 |
| Sodium Dodecyl Sulphonate (as active) | 1.03 | 1.25 | 1.25 | 0.95 |
| **Surfactant solution** | | | | |
| Deoxygenated Water | 181.0 | 220.7 | 221.7 | 167.2 |
| Sodium Dodecyl Sulphonate (as active) | 0.298 | 0.363 | 0.376 | 0.275 |
| Ammonium Persulfate (as active) | 0.073 | 0.089 | 0.089 | 0.068 |
| **Post Addition** | | | | |
| Ammonium Persulfate (as active) | 0.033 | 0.040 | 0.040 | 0.030 |
| [1]Weight average molecular weight 700g/mol. | | | | |

Example 1

Mica containing formulations as described in Table 2 were prepared with the above described copolymers,

Carbopol® 980, or Aculyn® 28 acrylate copolymer following the order of addition procedures described in Table 3. The amount of such copolymers is reported on an active basis. Sodium chloride was added to adjust viscosity to a target viscosity of 5500 to 7500 centipoise (cP) as measured by the viscosity procedure described below. The target pH to which the compositions were adjusted was 5.5-6.5, which was achieved by the addition of sodium hydroxide and/or citric acid. Example 13 was unstable; mica was observed to settle out of same.

Photographs of several of the compositions are provided in Figures 1, 2 and 3. Example 1 was a dull grey liquid. Examples 3, 6 and 7 (from Figures 1 and 2), and Examples 14 or 15 (from Figure 3) were all shinier than Example 2. Examples 4, 5 were similar to Example 2.

Spectrophotometric measurements were obtained from Examples 1, 2, 3, 5, and 6 using the procedure described below; results are reported in Table 4. Although the information provided by the spectrophotometric testing is limited in that the measurements are obtained at a single fixed angle on thin films, the combination of total specular reflectance and brightness offers information that provides a correlation with components of visual appearance. Of the six materials tested, Example 6 had the best visual appearance; it was the sample with the highest brightness and second highest total specular reflectance values. Example 3 had brightness and total specular reflectance values that were higher than that of Example 2, and was visually perceived as shinier. Example 8, which was visually perceived as shinier than Example 2, had a comparable total specular reflectance value, but a higher brightness value. Example 5, which was visually perceived as similar to Example 2 had a somewhat lower total specular reflectance value, but a much higher brightness value. Example 1, which had a dull grey appearance, had a total specular reflectance value that was much lower than that of the other five materials tested. Examples 14 and 15 were visually perceived as shinier than Example 2, and although Spectrometric measurements were not conducted, it would be expected that such measurements would corroborate enhanced visual shininess

The spectrophotometric data for Examples 3 and 5 (each of which contained 0.4wt.% of Copolymer A, and was prepared by order of addition procedure D) further shows that total specular reflectance for these samples increased with mica content, while brightness decreased. Notwithstanding that Example 5 had $1/3^{rd}$ the amount of mica as Example 2 (0.05wt.% compared to 0.15wt.%), it had a visual appearance that was similar. Example 3 (0.15% mica) was visually perceived as shinier than Example 2 (which contained 0.4 wt.% of Aculyn® 28 acrylate copolymer).

Viscosity Procedure

A Brookfield Viscometer was used to measure product viscosity. Viscosities are reported as cP at 25°C and 20 rpm (spindle 5).

Spectrophotometric Procedure

Spectrophotometric measurements are obtained in a wavelength range of 360 to 740nm (which approximates that of visible light), using a hand-held, calibrated spectrophotometer (Model CM-2600d from Konica Minolta Sensing Americas, Inc. or equivalent), which has the capability to operate in such wavelength range in both the SCI (specular component included) mode and SCE (specular component excluded) mode almost simultaneously. Measurements were taken using an aperture size of 8mm and a fixed measurement angle of 10°C.

Three 10cm x 16 mm film strips (75 microns thick) of the material to be measured are laid down on a black and white drawdown card (Form 2A Opacity Chart from The Leneta Company, or equivalent). Uniformity of film thickness can be obtained using, for example, a film applicator of suitable gap size; one such applicator is a1103:25mm cube film applicator (film width of 16 mm, 37/75 micron gap size) from Sheen Instruments, Ltd.

The films are dried in a controlled environment (temperature 22.2°C and 40% humidity) for 30 minutes prior to measuring same. Measurements (specular reflectance in the SCI mode and specular reflectance in the SCE mode) are taken from the black section of the card from 10 different locations on the film strips (at least 3 measurements from each strip, for a total of 10 samples).

Total specular reflection (G) is obtained by subtracting the spectral reflectance in SCE mode (i.e., the diffuse reflectance) from that in the SCI mode and integrating same across the wavelength range of the measurement (360 to 740nm), i.e.,

$$G = \int_{\lambda} (SCI - SCE) \cdot d\lambda$$

The integration of the diffuse reflectance across the wavelength range of measurement yields the diffuse component D:

$$D = \int_{\lambda} SCE \cdot d\lambda$$

The brightness value is calculated as G/D and is a measure of the contrast between the total specular reflection and the diffuse component D.

Results are reported as the average values (G or G/D) for the 10 samples.

TABLE 2

| **Ingredient (wt. %)** | Example1 (Comparative) | Example 2 (Comparative) | Example 3 | Example 4 | Example 5 | Example 6 | Example 7 |
|---|---|---|---|---|---|---|---|
| Order of Addition Procedure | A | A | D | A | D | E | G |
| Sodium Laureth Sulphate, 1 –EO Aqueous solution containing 28 wt. % active; herein abbreviated as SLES. | 12.0 | 12.0 | 12.0 | 12.0 | 12.0 | 12.0 | 12.0 |
| Cocoamidopropyl Betaine Aqueous solution containing 30 wt. % active; herein abbreviated as CAPB. | 1.6 | 1.6 | 1.6 | 1.6 | 1.6 | 1.6 | 1.6 |
| Guar Hydroxypropyltrimonium Chloride; herein abbreviated as Cationic Guar | 0.2 | 0,2 | 0.2 | 0.2 | 0.2 | 0.2 | 0.2 |
| Carbopol® 980 Crosslinked Acrylate Polymer | 0.4 | | | | | | |
| Aculyn® 28 Copolymer | | 0.4 | | | | | |
| Copolymer A | | | 0.4 | 0.4 | 0.4 | 0.4 | 0.4 |
| Copolymer B | | | | | | | |
| Copolymer C | | | | | | | |
| Timiron® Super Sheen MP1001 Mica | 0.15 | 0.15 | 0.15 | 0.15 | 0.05 | 0.15 | 0.15 |
| Timiron® Silverstar MP 2000 Mica | | | | | | | |
| Citric Acid (as needed for pH adjustment) | 0 – 0.1 | 0 – 0.1 | 0 – 0.1 | 0 – 0.1 | 0 – 0.1 | 0 – 0.1 | 0 – 0.1 |
| Sodium Hydroxide (50 wt. % aqueous soln.; as needed for pH adjustment) | 0.05 – 0.18 | 0.05 – 0.18 | 0.05 – 0.18 | 0.05 – 0.18 | 0.05 – 0.18 | 0.05 – 0.18 | 0.05 – 0.18 |
| Sodium Chloride (as needed for viscosity adjustment) | 0.3 – 0.65 | 0.3 – 0.65 | 0.3 – 0.65 | 0.3 – 0.65 | 0.3 – 0.65 | 0.3 – 0.65 | 0.3 – 0.65 |
| Wacker UL9815 Dimethiconol Emulsion | 1.7 | 1.7 | 1.7 | 1.7 | 1.7 | 1.7 | 1.7 |
| Fragrance | 0.7 | 0.7 | 0.7 | 0.7 | 0.7 | 0.7 | 0.7 |
| DMDM Hydantoin (aqueous solution containing 55 wt. % active) | 0.1 | 0.1 | 0.1 | 0.1 | 0.1 | 0.1 | 0.1 |
| Demineralized Water | To 100 | To 100 | To 100 | To 100 | To 100 | To 100 | To 100 |

**TABLE 2 (CONTINUED)**

| **Ingredient (wt. %)** | Example 14 | Example15 |
|---|---|---|
| Order of Addition Procedure | E | G |
| Sodium Laureth Sulphate, 1 –EO<br>Aqueous solution containing 28 wt. % active; herein abbreviated as SLES. | 12.0 | 12.0 |
| Aqueous solution containing 30 wt. % active; herein abbreviated as CAPB. | 1.6 | 1.6 |
| Guar Hydroxypropyltrimonium Chloride; herein abbreviated as Cationic Guar | 0.2 | 0.2 |
| Carbopol® 980<br>Crosslinked Acrylate Polymer | | |
| Aculyn® 28 Copolymer | | |
| Copolymer A | | |
| Copolymer B | | |
| Copolymer C | | |
| Copolymer D | 0.4 | 0.4 |
| Timiron® Supersheen MP1001 Mica | 0.15 | 0.15 |
| Timiron® Silverstar MP 2000 Mica | | |
| Citric Acid<br>(as needed for pH adjustment) | 0 - 01 | 0-0.1 |
| Sodium Hydroxide<br>(as needed for 50 wt. % aqueous soln.; for pH adjustment) | 0.05-0.18 | 0.05-0.18 |
| Sodium Chloride (as needed for viscosity adjustment) | 0.3-0.65 | 0.3-0.65 |
| Wacker UL9815<br>Dimethiconol Emulsion | 1.7 | 1.7 |
| Fragrance | 0.7 | 0.7 |
| DMDM Hydantoin<br>(aqueous solution containing 55 wt. % active) | 0.1 | 0.1 |
| Demineralized Water | To 100 | To 100 |

TABLE 3

| ORDER OF ADDITION PROCEDURE | | | | | | | | | | |
|---|---|---|---|---|---|---|---|---|---|---|
| Procedure | 1 | 2 | 3 | 4 | 5 | 6 | 7 | 8 | 9 | 10 |
| A | SLES + Water | Acrylate Copolymer | | | CATIONIC GUAR | CAPB | | | NaCl | NaOH |
| B | SLES + Water | Acrylate Copolymer | NaOH | | CATIONIC GUAR | CAPB | Citric acid | | NaCl | |
| C | SLES + Water | Acrylate Copolymer | NaOH | Citric acid | CATIONIC GUAR | CAPB | | | NaCl | |
| D | Water | Acrylate Copolymer | NaOH | SLES | CATIONIC GUAR | CAPB | Citric acid | | NaCl | |
| E | Water | Acrylate Copolymer | NaOH | SLES | | CAPB | Citric acid | CATIONIC GUAR | NaCl | |
| F | Water | Acrylate Copolymer | NaOH | SLES | CATIONIC GUAR | CAPB | NaCl | | Citric acid | |
| G | Water | Acrylate Copolymer | NaOH | SLES + NaCl | CATIONIC GUAR | CAPB | Citric acid | | | |

Fragrance was added together with the cationic guar as a premix.

Silicone was added before CAPB, and DMDM hydantoin was added after CAPB.

TABLE 4

| SPECTROPHOTOMETRIC MEASUREMENTS | | |
|---|---|---|
| Composition | Brightness (G/D) | Total Specular Reflectance (G) |
| Example 1 (Comparative) | 78 | 114 |
| Example 2 (Comparative) | 63 | 142 |
| 3 | 73 | 171 |
| 5 | 138 | 135 |
| 6 | 143 | 151 |

## Claims

1. A cleansing composition that comprises:

i) a copolymer that is the polymerization product of:

a) from 0.1 to 5 wt. % of a first ethylenically unsaturated monomer selected from:

a diacid of the formula:

$$HOOC\text{-}CR^1{=}CR^2\text{-}COOH \quad (I),$$

a cyclic anhydride precursor of diacid (I), the anhydride having the formula:

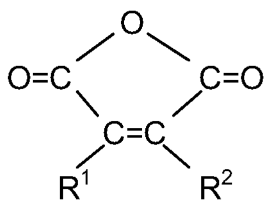

(II)

and combinations thereof,

wherein $R^1$ and $R^2$ are individually selected from H, $C_1$-$C_3$ alkyl, phenyl, chlorine and bromine,
b) from 15 to 60 wt. % of a second ethylenically unsaturated monomer selected from acrylic acid, methacrylic acid and combinations thereof,
c) from 30 to 75 wt. % of a (meth)acrylate monomer selected from $C_1$ to $C_8$ alkyl esters of (meth)acrylic acid and combinations thereof,
d) from 1 to 25wt.% of an associative monomer of the formula:

$$R^4\text{-}CH{=}C(R^3)\text{-}C(O)\text{-}O\text{-}(R^5O)_a\text{-}R^6 \quad (III)$$

wherein:

$R^3$ and $R^4$ are independently selected from H and $C_{1-3}$ alkyl,
each $R^5$ is independently an alkylene unit having from 2 to 4,
$R^6$ is selected from:

linear and branched alkyl having from 8 to 40 carbon atoms, and
alkaryl, the alkyl group of which has from 8 to 40 carbon atoms, such alkyl group being linear or

branched; and

a is from 6 to 40;
wherein the copolymer is at least partially deprotonated;

ii) cleansing surfactant;
iii) reflective particles; and
iv) water.

**2.** The cleansing compositions according to claim 1 wherein the first ethylenically unsaturated monomer is selected from maleic acid, fumaric acid, citraconic acid, maleic anhydride, citraconic anhydride, and combinations thereof.

**3.** The cleansing composition according to claim 1 wherein maleic anhydride or maleic acid accounts for at least 95 % by weight of the total weight of the first ethylenically unsaturated monomer.

**4.** The cleansing composition according to claim 1 wherein the ratio, by weight, of first ethylenically unsaturated monomer (a) to associative monomer (d) is from 1:5 to 1:22.

**5.** The cleansing composition according to claim 2 wherein methacrylic acid provides at least 50 % by weight of the total weight of the second ethylenically unsaturated monomer.

**6.** The cleansing composition according to claim 2 wherein the associative monomer has the structure:

$$CH_3[CH_2]_b\text{-}CH_2\text{-}[OCH_2CH_2]_a\text{-}O\text{-}C(O)C(R^3)\text{=}CHR^4 \qquad \text{IV}$$

wherein b is from 8 to 28 and a is from 15 to 35.

**7.** The cleansing composition according to claim 6 wherein the (meth)acrylate monomer is selected from $C_1$ to $C_4$ alkyl esters of acrylic acid, $C_1$ to $C_4$ alkyl esters of methacrylic acid and combinations thereof.

**8.** The cleansing composition according to claim 7 wherein $C_1$ to $C_4$ alkyl ester of acrylic acid provides at least 90 % by weight of the total weight of the meth(acrylate) monomer.

**9.** The cleansing composition according to claim 8 wherein the first ethylenically unsaturated monomer is selected from maleic acid, maleic anhydride and combinations thereof.

**10.** The cleansing composition according to claim 8 wherein said maleic acid, maleic anhydride or mixtures accounts for at least 95 % by weight of the total weight of the first ethylenically unsaturated monomer, $R^3$ is methyl and R is hydrogen.

**11.** The cleansing composition according to claim 1 wherein the reflective particles comprise mica.

**12.** The cleansing composition according to claim 1 wherein the cleansing surfactant comprises anionic surfactant.

**13.** The cleansing composition according to claim 1 wherein the cleansing surfactant composition further comprises amphoteric surfactant.

**14.** The cleansing composition according to claim 1 further comprising a silicone conditioning agent.

**15.** The cleansing composition according to claim 1 wherein the reflective particles are present in an amount of from 0.1 to 0.4 wt. %, based on the total weight of the composition.

**16.** The cleansing composition according to claim 1 wherein the reflective particles are present in an amount of 0.05 to 0.2 wt. %, based on the total weight of the composition.

**17.** The cleansing composition according to claim 1 wherein the structured liquid has a micellar structure.

**18.** A cleansing composition according to claim 1 wherein the copolymer (i) is sufficiently neutralized that is becomes

substantially completely deprotonated.

**19.** A composition according to claim 1, wherein said copolymer is at least 70% neutralized.

**20.** A composition according to claim 18 wherein the copolymer achieves at least 90% maximum viscosity at the concentration at which it is present.

**21.** A composition according to claim 20 which is fully neutralized and achives maximum viscosity at the concentration at which it is present.

**22.** A composition according to claim 1, wherein the copolymer is treated with neutralizing agent prior to the composition being formed into structured liquid.

**23.** The cleansing composition according to claim 1 wherein copolymer (i) is a linear, non-crosslinked polymer.

**24.** The cleansing composition according to claim 1 wherein the composition has a brightness value of at least 70 and a total specular reflectance value of at least 150, and contains reflective particles in an amount up to 0.2 wt.%, based on the total weight of the composition.

**Patentansprüche**

**1.** Reinigungszusammensetzung, die umfasst:

i) ein Copolymer, das das Polymerisationsprodukt ist von:

a) von 0,1 bis 5 Gew.-% eines ersten ethylenisch ungesättigten Monomers, ausgewählt aus:

einer Disäure der Formel:

$$HOOC\text{-}CR^1\text{=}CR^2\text{-}COOH \qquad (I),$$

einem cyclischen Anhydrid-Vorläufer der Disäure (I), wobei das Anhydrid die Formel:

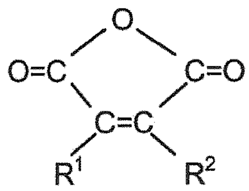

(II)

aufweist,
und Kombinationen davon,
wobei $R^1$ und $R^2$ individuell aus H, $C_{1-3}$-Alkyl, Phenyl, Chlor und Brom ausgewählt sind,

b) von 15 bis 60 Gew.-% eines zweiten ethylenisch ungesättigten Monomers, ausgewählt aus Acrylsäure, Methacrylsäure und Kombinationen davon,
c) von 30 bis 75 Gew.-% eines (Meth)acrylat-Monomers, ausgewählt aus $C_1$- bis $C_8$-Alkylestern von (Meth)acrylsäure und Kombinationen davon,
d) von 1 bis 25 Gew.-% eines assoziativen Monomers der Formel:

$$R^4\text{-}CH\text{=}C(R^3)\text{-}C(O)\text{-}O\text{-}(R^5O)_a\text{-}R^6 \qquad (III),$$

wobei:

$R^3$ und $R^4$ unabhängig voneinander aus H und $C_{1-3}$-Alkyl ausgewählt sind,
wobei jedes $R^5$ unabhängig eine Alkylen-Einheit, die 2 bis 4 aufweist,

darstellt,
$R^6$ ausgewählt ist aus:

linearem und verzweigtem Alkyl mit von 8 bis 40 Kohlenstoffatomen und Alkaryl, wobei die Alkylgruppe hiervon von 8 bis 40 Kohlenstoffatome aufweist, wie eine Alkylgruppe, die linear oder verzweigt ist, und
a von 6 bis 40 ist,
wobei das Copolymer mindestens partiell deprotoniert ist,

ii) Reinigungstensid,
iii) reflektierende Partikel und
iv) Wasser.

**2.** Reinigungszusammensetzung nach Anspruch 1, wobei das erste ethylenisch ungesättigte Monomer aus Maleinsäure, Fumarsäure, Citraconsäure, Maleinsäureanhydrid, Citraconsäureanhydrid und Kombinationen davon ausgewählt ist.

**3.** Reinigungszusammensetzung nach Anspruch 1, wobei das Maleinsäureanhydrid oder die Maleinsäure mindestens 95 Gewichts-% des Gesamtgewichts des ersten ethylenisch ungesättigten Monomers ausmachen.

**4.** Reinigungszusammensetzung nach Anspruch 1, wobei das Verhältnis, in Gewicht, des ersten ethylenisch ungesättigten Monomers (a) zum assoziativen Monomer (d) von 1:5 bis 1:22 beträgt.

**5.** Reinigungszusammensetzung nach Anspruch 2, wobei die Methacrylsäure mindestens 50 Gewichts-% des Gesamtgewichts des zweiten ethylenisch ungesättigten Monomers bereitstellt.

**6.** Reinigungszusammensetzung nach Anspruch 2, wobei das assoziative Monomer die Struktur

$$CH_3[CH_2]_b\text{-}CH_2\text{-}[OCH_2CH_2]_a\text{-}O\text{-}C(O)C(R^3)\text{=}CHR^4 \qquad \text{IV}$$

aufweist,
wobei b von 8 bis 28 und a von 15 bis 35 beträgt.

**7.** Reinigungszusammensetzung nach Anspruch 6, wobei das (Meth)acrylat-Monomer aus $C_1$- bis $C_4$-Alkylestern von Acrylsäure, $C_1$- bis $C_4$-Alkylestern von Methacrylsäure und Kombinationen davon ausgewählt ist.

**8.** Reinigungszusammensetzung nach Anspruch 7, wobei der $C_1$- bis $C_4$-Alkylester der Acrylsäure mindestens 90 Gewichts-% des Gesamtgewichts des Meth(acrylat)-Monomers bereitstellt.

**9.** Reinigungszusammensetzung nach Anspruch 8, wobei das erste ethylenisch ungesättigte Monomer aus Maleinsäure, Maleinsäureanhydrid und Kombinationen davon ausgewählt ist.

**10.** Reinigungszusammensetzung nach Anspruch 8, wobei die Maleinsäure, das Maleinsäureanhydrid oder Mischungen davon mindestens 95 Gewichts-% des Gesamtgewichts des ersten ethylenisch ungesättigten Monomers, wobei $R^3$ Methyl und R Wasserstoff ist, ausmachen.

**11.** Reinigungszusammensetzung nach Anspruch 1, wobei die reflektierenden Partikel Glimmer umfassen.

**12.** Reinigungszusammensetzung nach Anspruch 1, wobei das Reinigungstensid anionisches Tensid umfasst.

**13.** Reinigungszusammensetzung nach Anspruch 1, wobei die Reinigungstensidzusammensetzung ferner amphoteres Tensid umfasst.

**14.** Reinigungszusammensetzung nach Anspruch 1, die des Weiteren ein Silikon-Konditionierungsmittel umfasst.

**15.** Reinigungszusammensetzung nach Anspruch 1, wobei die reflektierenden Partikel in einer Menge von 0,1 bis 0,4 Gew.-%, bezogen auf das Gesamtgewicht der Zusammensetzung, vorliegen.

**16.** Reinigungszusammensetzung nach Anspruch 1, wobei die reflektierenden Partikel in einer Menge von 0,05 bis 0,2 Gew.-%, bezogen auf das Gesamtgewicht der Zusammensetzung, vorliegen.

**17.** Reinigungszusammensetzung nach Anspruch 1, wobei die strukturierte Flüssigkeit eine mizellare Struktur aufweist.

**18.** Reinigungszusammensetzung nach Anspruch 1, wobei das Copolymer (i) ausreichend neutralisiert ist, so dass es im Wesentlichen vollständig deprotoniert wird.

**19.** Zusammensetzung nach Anspruch 1, wobei das Copolymer zu mindestens 70% neutralisiert ist.

**20.** Zusammensetzung nach Anspruch 18, wobei das Copolymer mindestens 90% maximale Viskosität bei der Konzentration, bei der es vorliegt, erreicht.

**21.** Zusammensetzung nach Anspruch 20, welche vollständig neutralisiert ist und maximale Viskosität bei der Konzentration, bei der sie vorliegt, erreicht.

**22.** Zusammensetzung nach Anspruch 1, wobei das Copolymer mit einem Neutralisationsmittel behandelt wird, bevor die Zusammensetzung in eine strukturierte Flüssigkeit umgeformt wird.

**23.** Reinigungszusammensetzung nach Anspruch 1, wobei das Copolymer (i) ein lineares, nicht-vernetztes Polymer darstellt.

**24.** Reinigungszusammensetzung nach Anspruch 1, wobei die Zusammensetzung einen Helligkeitswert von mindestens 70 und eine gesamte Spiegelreflektion von mindestens 150 aufweist und reflektierende Partikel in einer Menge bis zu 0,2 Gew.-%, bezogen auf das Gesamtgewicht der Zusammensetzung, enthält.

## Revendications

**1.** Composition nettoyante qui comprend :

i) un copolymère qui est le produit de polymérisation de :

a) de 0,1 à 5 % en masse d'un premier monomère éthyléniquement insaturé choisi parmi :

un diacide de la formule :

$$HOOC\text{-}CR^1\text{=}CR^2\text{-}COOH \qquad (I),$$

un précurseur d'anhydride cyclique de diacide (I), l'anhydride ayant la formule :

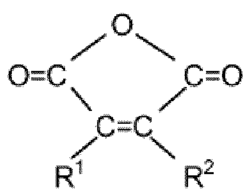

(II)

et des combinaisons de ceux-ci,
où $R^1$ et $R^2$ sont individuellement choisis parmi H, un groupe alkyle en $C_1$-$C_3$, phényle, chlore et brome,

b) de 15 à 60 % en masse d'un second monomère éthyléniquement insaturé choisi parmi l'acide acrylique, l'acide méthacrylique et des combinaisons de ceux-ci,
c) de 30 à 75 % en masse d'un monomère de (méth)acrylate choisi parmi des esters alkyliques en $C_1$ à $C_8$ d'acide (méth)acrylique et des combinaisons de ceux-ci,
d) de 1 à 25 % en masse d'un monomère associatif de la formule :

$$R^4\text{-}CH\text{=}C(R^3)\text{-}C(O)\text{-}O\text{-}(R^5O)_a\text{-}R^6 \qquad (III)$$

où :

$R^3$ et $R^4$ sont indépendamment choisis parmi H et un groupe alkyle en $C_{1-3}$,
chaque $R^5$ est indépendamment une unité alkylène ayant de 2 à 4,
$R^6$ est choisi parmi :

un groupe alkyle linéaire et ramifié ayant de 8 à 40 atomes de carbone, et alkaryle, dont le groupe alkyle présente de 8 à 40 atomes de carbone, tel qu'un groupe alkyle linéaire ou ramifié ; et

a est de 6 à 40 ;
où le copolymère est au moins partiellement déprotoné ;

ii) un tensioactif nettoyant ;
iii) des particules réfléchissantes ; et
iv) de l'eau.

**2.** Composition nettoyante selon la revendication 1, où le premier monomère éthyléniquement insaturé est choisi parmi l'acide maléique, l'acide fumarique, l'acide citraconique, l'anhydrique maléique, l'anhydride citraconique, et des combinaisons de ceux-ci.

**3.** Composition nettoyante selon la revendication 1, où l'anhydride maléique ou l'acide maléique compte pour au moins 95 % en masse de la masse totale du premier monomère éthyléniquement insaturé.

**4.** Composition nettoyante selon la revendication 1, où le rapport, en masse, de premier monomère éthyléniquement insaturé (a) à monomère associatif (d) est de 1:5 à 1:22.

**5.** Composition nettoyante selon la revendication 2, où l'acide méthacrylique fournit au moins 50 % en masse de la masse totale du second monomère éthyléniquement insaturé.

**6.** Composition nettoyante selon la revendication 2, où le monomère associatif présente la structure :

$$CH_3[CH_2]_b\text{-}CH_2\text{-}[OCH_2CH_2]_a\text{-}O\text{-}C(O)C(R^3)\text{=}CHR^4 \qquad \text{IV}$$

où b est de 8 à 28 et a est de 15 à 35.

**7.** Composition nettoyante selon la revendication 6, où le monomère de (méth)acrylate est choisi parmi des esters alkyliques en $C_1$ à $C_4$ d'acide acrylique, des esters alkyliques en $C_1$ à $C_4$ d'acide méthacrylique et des combinaisons de ceux-ci.

**8.** Composition nettoyante selon la revendication 7, où l'ester alkylique en $C_1$-$C_4$ d'acide acrylique fournit au moins 90 % en masse de la masse totale du monomère de (méth)acrylate.

**9.** Composition nettoyante selon la revendication 8, où le premier monomère éthyléniquement insaturé est choisi parmi l'acide maléique, l'anhydride maléique et des combinaisons de ceux-ci.

**10.** Composition nettoyante selon la revendication 8, où ledit acide maléique, anhydride maléique ou mélange compte pour au moins 95 % en masse de la masse totale du premier monomère éthyléniquement insaturé, $R^3$ est le groupe méthyle et R est l'hydrogène.

**11.** Composition nettoyante selon la revendication 1, où les particules réfléchissantes comprennent du mica.

**12.** Composition nettoyante selon la revendication 1, où le tensioactif nettoyant comprend un tensioactif anionique.

**13.** Composition nettoyante selon la revendication 1, où la composition de tensioactif nettoyante comprend de plus un tensioactif amphotère.

**14.** Composition nettoyante selon la revendication 1 comprenant de plus un agent de conditionnement de silicone.

15. Composition nettoyante selon la revendication 1, où les particules réfléchissantes sont présentes dans une quantité de 0,1 à 0,4 % en masse, rapporté à la masse totale de la composition.

16. Composition nettoyante selon la revendication 1, où les particules réfléchissantes sont présentes dans une quantité de 0,05 à 0,2 % en masse, rapporté à la masse totale de la composition.

17. Composition nettoyante selon la revendication 1, où le liquide structuré présente une structure micellaire.

18. Composition nettoyante selon la revendication 1, où le copolymère (i) est suffisamment neutralisé qu'il devient pratiquement complètement déprotoné.

19. Composition selon la revendication 1, où ledit copolymère est neutralisé à au moins 70 %.

20. Composition selon la revendication 18, où le copolymère réalise au moins 90 % de viscosité maximale à la concentration à laquelle il est présent.

21. Composition selon la revendication 20, qui est complètement neutralisée et réalise une viscosité maximale à la concentration à laquelle elle est présente.

22. Composition selon la revendication 1, où le copolymère est traité avec un agent neutralisant avant que la composition soit formée en liquide structurel.

23. Composition nettoyante selon la revendication 1, où le copolymère (i) est un copolymère linéaire, non réticulé.

24. Composition nettoyante selon la revendication 1, où la composition présente une valeur de brillance d'au moins 70 et une valeur de réflexion spéculaire totale d'au moins 150, et contient des particules réfléchissantes dans une quantité jusqu'à 0,2 % en masse, rapporté à la masse totale de la composition.

FIGURE 1

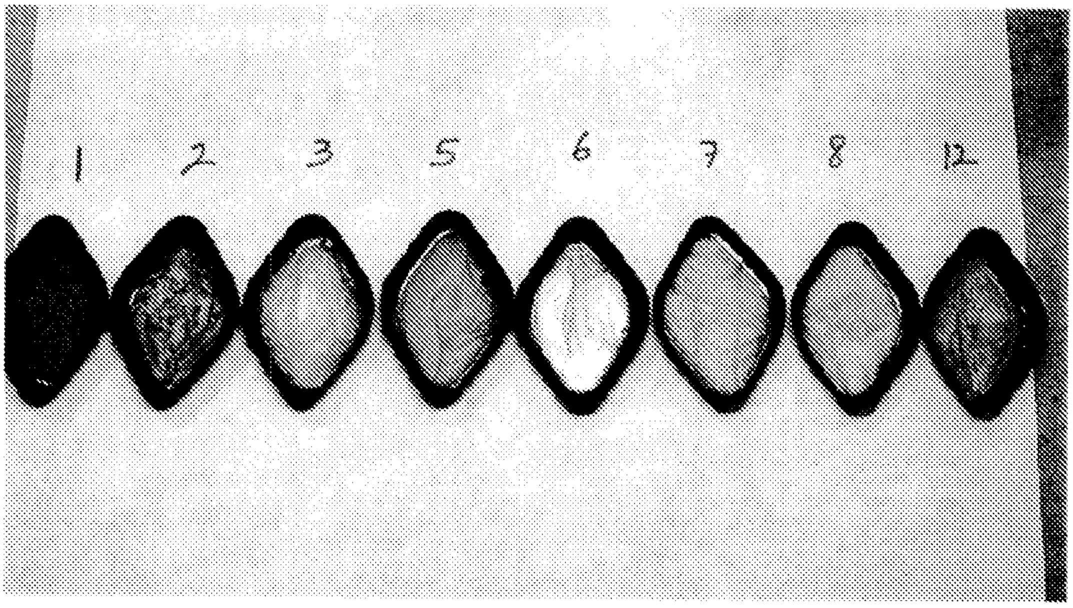

FIGURE 2

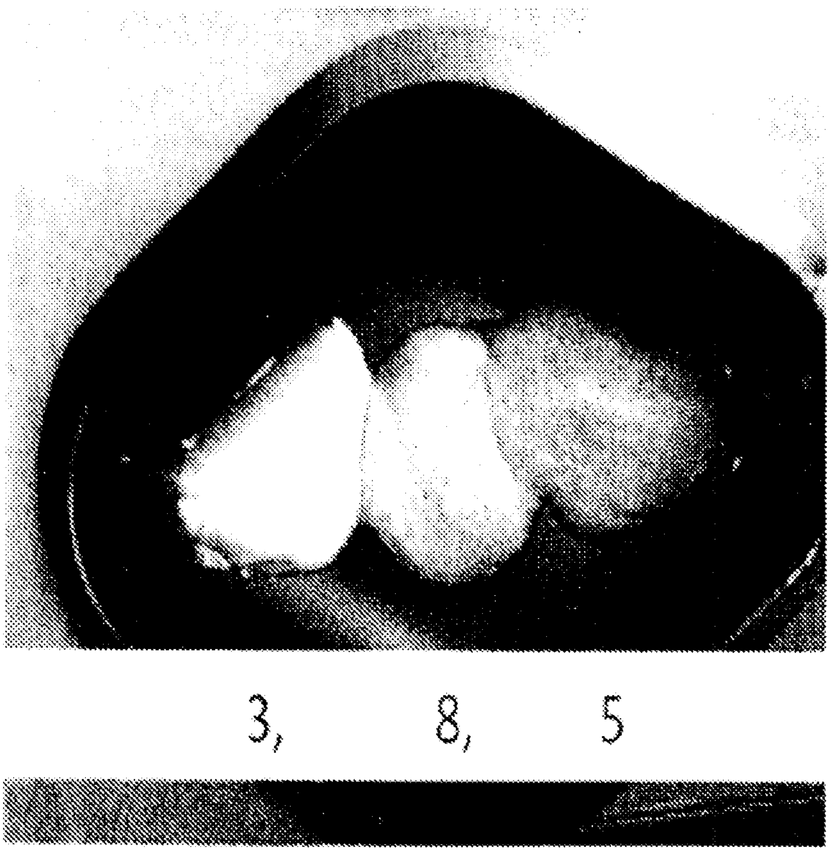

FIGURE 3

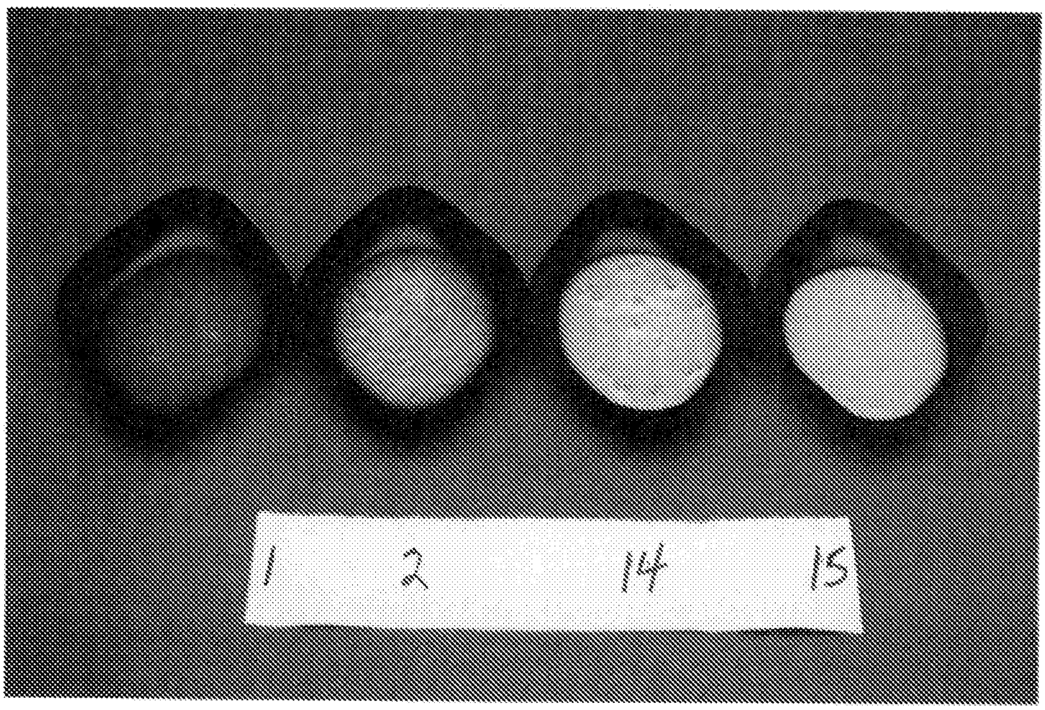

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4529773 A, Witiak
- US 7649047 B
- US 7288616 B, Tamareselvy
- US 7378479 B, Tamareselvy
- US 7217752 B, Schmucker-Castner
- WO 2010026097 A1, Graham
- WO 2007090759 A
- EP 2012068732 W
- WO 9206154 A
- US 5194639 A
- US 8268767 B
- WO 9631188 A
- EP 0530974 A